# EUROPEAN PATENT APPLICATION

(11) **EP 3 473 338 A1**
(43) Date of publication of application: **24.04.2019**
(21) Application number: 17197242.5
(22) Date of filing: 19.10.2017
(51) Int. Cl.: B01L 3/00, A61B 10/00, G01N 35/00

(54) **A MICROFLUIDIC CARTRIDGE AND SAMPLE CARRIER**

(71) Applicant: Koninklijke Philips N.V., 5656 AE Eindhoven (NL)
(72) Inventor: WIMBERGER-FRIEDL, Reinhold, 5656 AE Eindhoven (NL); SCHUCK, Jochen, 5656 AE Eindhoven (NL); PIERIK, Anke, 5656 AE Eindhoven (NL)
(74) Representative: de Haan, Poul Erik

(57) **Abstract**

In the field of tissue sample extraction, a barrier layer may be applied on top of a sample of biological material on a sample slide to expose portions of the biological material which are of interest in a tissue sample extraction process, and to seal portions of the biological material which is not desired in a tissue sample extraction process.

The present aspect describes an approach enabling predefined local extraction of biomolecules from a biological sample. It is proposed to perform sample processing and detection using an integrated cartridge and a sample carrier by providing a seal around an aperture exposing biological material on the sample carrier to thus form a sealable analysis chamber between the integrated cartridge and the sample carrier.

## Description

### FIELD OF THE INVENTION

The invention relates to a microfluidic cartridge and sample carrier for predefined local extraction of biomolecules from biological sample. It also relates to a method, a system, and a kit of parts for the predefined local extraction of biomolecules from a biological sample.

### BACKGROUND OF THE INVENTION

Conventionally, a method of tissue pathology involves a lab technician dissecting selected area of tissue from a consecutive tissue section and providing it to a molecular analysis lab, where an analysis is carried out.

WO 2014/054016 A1 discusses a method and sample isolation unit for isolating a sample region of interest in a sample by exposing positions outside the sample region of interest to a light beam such that the sample material is altered to become separable from the remainder. This tissue alteration may particularly comprise the ablation of sample material. The light beam is preferably a laser light beam that is scanned across the sample. Following tissue separation, the sample material may be removed by a medical professional and used in a subsequent extraction process. However, the methods discussed in this document may be further improved.

### SUMMARY OF THE INVENTION

It is therefore an object of the invention to provide means that allow for a simplified isolation and analysis of a region of interest from sample.

According to a first aspect, there is provided a microfluidic cartridge for predefined local extraction of biomolecules from a biological sample. The microfluidic cartridge comprises a receptacle for receiving a sample carrier in an analysis position, wherein the receptacle comprises a sealable surface having a predefined geometry configured to match to a predefined barrier layer of a sample carrier placed in an analysis position, such that when the sample carrier is in the analysis position, an atmospherically closed connection is formed between the barrier layer and the sealable surface enclosing an aperture in the barrier layer around a region of interest of biological material in a sealable analysis chamber.

The microfluidic cartridge also comprises an extraction fluid storage chamber for storing an extraction fluid having at least one channel in fluid communication with the sealable analysis chamber, wherein the extraction fluid is used to release biomolecules from the region of interest of the biological sample, and an output portion to enable the transfer of extraction fluid comprising released biomolecules.

An effect is that a biomolecular extraction process may be performed directly from the sample carrier used to mount a sample of biological material, thus providing a closed and automated interface between the sample dissection and/or selection process, and the molecular analysis process. This removes the need for an unnecessary mechanical transfer of pieces of tissue, because the liquid biomolecular extraction process is integrated in the microfluidic cartridge.

Optionally, the microfluidic cartridge further comprises a fluid analysis chamber in fluid communication with the output portion. The fluid analysis chamber is in fluid communication with one or more of the analysers selected from the group of: a sample purifier using a filter or microbeads, a biomolecular amplifier configured to apply a primer to the released biomolecules, and fluorescence and/or electrical analysers.

An effect is that results of the biomolecular analysis of a sample of biological material on the sample carrier may be provided directly from the microfluidic cartridge as an analogue or digital electronic signal, for example.

Optionally, the fluid storage chamber and/or the fluid receiving chamber are in fluid connection with a port or ports located on an external surface of the microfluidic cartridge, to enable extraction fluid comprising released biomolecules to be transferred for external analysis.

An effect is that reagent containing biomolecules extracted from a predefined area of the sample slide may be efficiently conducted to an external analysis machine.

Optionally, the sealable surface of the receptacle is formed from a resilient substance selected from the group of rubber, foam rubber, silicon rubber, polyolefin, thermoplastic polymer, and EDPM.

An effect is that during predefined local extraction of biomolecules, reagent cannot escape from the sealable analysis chamber, and external contaminants are separated for the duration of the biomolecular extraction, thus leading to more accurate test results. The chance of cross-contamination between samples is reduced.

Optionally, the sealed analysis chamber has an internal volume in the range of 10-1000 µL.

Optionally, the receptacle further comprises a first actuator causing the sealable surface to be configurable into a first retracted position, and a first sealed position, and wherein the sealed extraction chamber is formed when the first actuator is actuated from the first retracted position into the first sealed position, to thus cause the sealable surface to move into a sealing engagement with a barrier layer of a sample carrier.

An effect is that the sample carrier is, from an unengaged position, initially engaged with the sealable surface of the microfluidic cartridge, and then progressively brought into a tighter-sealed position with respect to the microfluidic cartridge.

Optionally, the receptacle further comprises a sample carrier actuator causing a sample carrier, when held in the receptacle, to be configurable into a second retracted position, and a second sealed position, and wherein the sealed extraction chamber is formed when the sample carrier actuator is actuated from the second retracted position into the second sealed position, to thus cause a barrier layer of a sample carrier to move into a sealing engagement with the sealable surface of the receptacle.

An effect is that the sample carrier is, from an unengaged position, initially engaged with the sealable surface of the microfluidic cartridge, and then progressively brought into a tighter-sealed position with respect to the microfluidic cartridge

Optionally, the microfluidic cartridge further comprises a microfluidic cartridge carrier ID configured to identify the microfluidic cartridge.

An effect is that a microfluidic analysis system may read different types of microfluidic cartridge, and ensure that the correct electronic and/or chemical reading protocol is formed, based on the specific test intended to be applied by the specific microfluidic cartridge.

According to a second aspect, there is provided a sample carrier for local extraction ofbiomolecules from a biological sample. The sample carrier comprises:
- a sample carrier configured to hold a biological sample, and
- an impermeable barrier layer covering a portion of the biological sample, and exposing a region of interest of the biological sample through an aperture in the barrier layer.

The barrier layer on the sample carrier has a predefined geometry which is configured to match to a predefined geometry of a sealable surface of a microfluidic cartridge such that when the sample carrier is in an analysis position of a microfluidic cartridge for predefined local extraction of biomolecules from a biological sample, a sealed analysis chamber is formed containing the region of interest of the biological sample.

An effect is that predefined region of biological material may be analysed on a sample carrier in the microfluidic cartridge without further mechanical distortion or biological contamination. The outer extent (dimensions) of the barrier layer on the sample carrier are fixed to ensure that the tissue is always inside a certain area of the sealable surface of the microfluidic cartridge. Around that, the sealable surface can be positioned, having a predefined geometry.

Optionally, the barrier layer of the sample carrier is formed from a layer of cured chemical applied to the sample carrier and a first area of the biological sample.

An effect is that the sealable barrier can be fabricated on the sample carrier.

Optionally, the cured chemical is selected from the group of: UV-cured resins, acrylates, methacrylate, enols, epoxides, and acrylamides.

An effect is that complicated aperture shapes in the sealable barrier layer may be provided, enabling the reliable analysis of regions of interest in the biological material.

According to a third aspect, there is provided a microfluidic analysis system, comprising:
- a cartridge receptacle for receiving a microfluidic cartridge according to the first aspect, an extraction fluid analyser and/or electronic interface configured to interface with the output portion of the microfluidic analysis system, and a data processor configured to generate analysis data based on an output of the extraction fluid analyser and/or the electronic interface and an output display configured to display microfluidic analysis results to a user.

According to a fourth aspect, there is provided a method for predefined local extraction of biomolecules from a biological sample. The method comprises:
a) receiving a sample carrier into an analysis position of a receptacle of a microfluidic cartridge, wherein the receptacle comprises a sealable surface having a predefined geometry configured to match to a predefined geometry of a barrier layer of a sample carrier placed in the analysis position, such that when the carrier is in the analysis position, an atmospherically closed connection is formed between the barrier layer and the sealable surface, enclosing an aperture in the barrier layer around a region of interest of biological material in a sealed analysis chamber;
b) transferring an extraction fluid into the sealed analysis chamber, wherein the extraction fluid is used to release biomolecules from the region of interest of the biological sample not covered by the barrier layer; and
c) outputting extraction fluid comprising biomolecules released from the region of interest of the biological sample not covered by the barrier layer to an output portion of the microfluidic cartridge.

Optionally, there is provided the step of:
b1) heating the sealed analysis chamber thereby to heat the extraction fluid in the sealed analysis chamber.

According to a fifth aspect, there is provided a kit of parts, comprising a microfluidic cartridge as defined according to the first aspect, and a sample carrier according to the second aspect.

According to an embodiment, there is provided a microfluidic cartridge according to the first aspect, or any of its embodiments, with a sample carrier according to the second aspect, or any of its embodiments, inserted into the receptacle of the microfluidic cartridge, such that the sealable surface of the microfluidic cartridge seals the region of interest on the sample carrier.

In the following application, the term "microfluidic cartridge" means a carrier having an integrated apparatus for the extraction of biomolecules which is optionally disposable. The microfluidic cartridge can be provided, for example, as an optionally hand-holdable injection-moulded plastic shell, with fluid channels, storage chambers and diaphragm pumps comprised within. The cartridge may optionally be thought of as an adapter, able to receive a sample carrier exposing a predefined region of biological sample, and to transmit the results of a biomolecular extraction process by electronic or chemical means to a microfluidic analysis system. No mechanical transfer of undefined pieces of biological material is necessary, because the liquid extraction process is integrated in the microfluidic cartridge.

In the following application, the term "predefined local extraction" means a sample of biological material is exposed to a fluid reagent (for example a lysing fluid) via an aperture in a sealable barrier layer applied onto regions of the biological material. For example, in a sample of biological material comprising a region with tumour tissue and a region with normal tissue, the sealable barrier layer is applied to a region having the tumour tissue. This may be performed, for example, by identifying a region having the tumour tissue in a biological sample using image processing, and subsequently providing commands to a sealable barrier layer generation unit (for example, a UV-curable inkjet printing unit) to provide a sealable barrier layer in regions of the biological sample having normal tissue, and to provide an aperture in the sealable barrier layer in regions of the biological sample having tumour tissue.

In the following application, the term "atmospherically closed" means a space which is entirely, or substantially fluid-tight at the range of pressures experienced in microfluidic analysis, for example pressures ranging up to 2000 kPa.

In the following application, the term "sealed" or "seal" implies that a microfluidic cartridge and sample carrier have been sealed from the external environment to enable a fluidic analysis in the cartridge. However, the term does not exclude that the region of interest on the sample carrier is not accessible, for example, by analysis fluids stored in the microfluidic cartridge.

It is, thus, a basic idea of the present application to make predefined local extraction of biomolecules simpler, more accurate and more reliable by applying the process of liquid sample extraction directly from a sample slide with a barrier layer, to thus provide a closed and automated interface between the sample dissection and selection process, and the molecular analysis process. This removes the need for mechanical transfer of pieces of tissue (for example, using tweezers or scalpels), and integrates the liquid extraction process in the sample preparation and detection device as part of a cartridge solution. Engagement of the sample carrier with the microfluidic device forms a sealed sample analysis chamber. Reagents are pumped from the cartridge reservoir to the chamber. Heating and temperature control devices are optionally provided to maintain the temperature of the buffer at the sample slide. Optionally, the buffer can be circulated. Because the region of interest is defined by the aperture in the barrier layer of the sample slide, which is applied in a separate step before attaching the sample slide into the cartridge, the buffer volume and process is independent of the size of the region of interest, and the thickness of the tissue section. Because the cartridges are disposable, cross-contamination between samples is reduced.

These, and other, aspects of the invention will be apparent from and elucidate it with reference to the following figures and the embodiments described hereinafter.

### BRIEF DESCRIPTION OF THE DRAWINGS

Figure 1 illustrates the provision of a sealable barrier layer on a sample carrier.
Figure 2 illustrates a general view of a microfluidic cartridge and sample carrier in accordance with aspects discussed herein.
Figure 3a) illustrates a top-down view of a sample carrier having a sample and a sealable barrier layer.
Figure 3b) illustrates a side-view of a sample carrier having a sample and sealable barrier layer.
Figure 3c) illustrates an exemplary plan view of predefined geometries of a seal region of a sample carrier and a sealable surface of a microfluidic cartridge.
Figure 4 illustrates an exemplary schematic cross-sectional view of a microfluidic cartridge with a sample carrier sealingly held within.
Figure 5a) illustrates an exemplary cross-sectional view of a microfluidic cartridge with a sample carrier sealingly held within prior to an extraction step.
Figure 5b) illustrates an exemplary cross-sectional view of a microfluidic cartridge with a sample carrier sealingly held within during an extraction step.
Figure 5c) illustrates an exemplary cross-sectional view of a microfluidic cartridge with a sample carrier sealingly held within after the completion of an extraction step.
Figure 6 illustrates a schematic view of a microfluidic analysis system in accordance with the third aspect discussed herein.

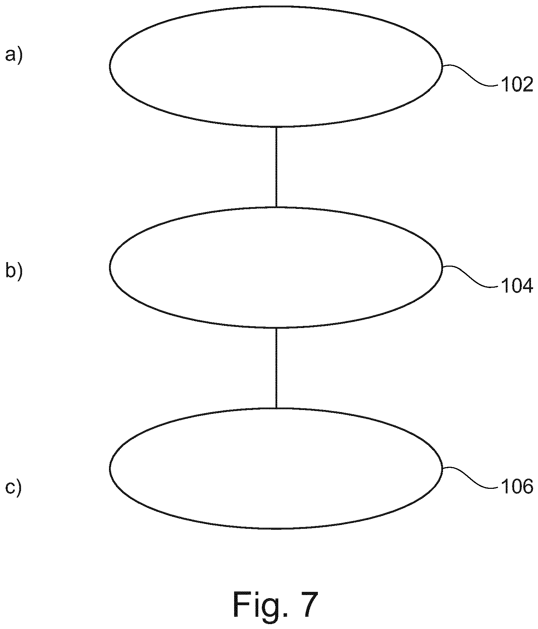

Figure 7 illustrates a method according to the fourth aspect as discussed herein.

### DETAILED DESCRIPTION OF EMBODIMENTS

Conventional molecular analysis of tissue, for example tumour tissue, is performed by a pathologist who selects a tumour sample for further analysis. For example, a region of a stained tissue slide containing tumour tissue is selected. A lab technician then dissects the area selected by the pathologist from a consecutive tissue section, and provides it (for example, in an Eppendorf tube) to a molecular analysis laboratory where the tissue analysis is carried out. Manual dissection of tissue from an area of interest can lead to uncontrolled, user-dependent, quality compromises. Manual dissection is also labour-intensive and difficult to standardise, leading to a situation where the quality of the sample undergoing molecular testing is largely undefined.

Following dissection, the tissue sample undergoes a process to extract relevant biomolecules by exposing the tissue to a lysis buffer (reagent), typically at an elevated temperature, for a certain incubation time. The lysis buffer containing extracted biomolecules may then be provided to a molecular laboratory for further processing.

Next-generation sequencing techniques will demand more attention to sample quality, because the process of tissue sample selection is often carried out in batch mode to improve efficiency. Tissue samples are collected over an entire week, and processed on a fixed day. Presently, therefore, total analysis times are between one and two weeks. So-called "random-access platforms" for molecular analysis can reduce such a time interval, but this implies that the sample dissection process must be available as a random access solution as well. The open-air and manual processing of tissue samples from one process to the next can lead to cross-contamination via the air, bench surface, or tools used.

The molecular analysis stage can be provided by an integrated sample preparation system that can process a dissected tissue sample using a robot, or a closed cartridge with microfluidic manipulation comprising amplification and detection of biomarkers.

Semi-automated tissue sample preparation techniques have been proposed. For example, WO 2014/054016 A1 discusses a tissue sample separation system using laser ablation. WO 2014/184005 A1 discusses the provision of a structured cover sheet having an aperture at a region of interest of a tissue sample.

However, a semi-automated tissue sample preparation technique means that a tissue sample must still ultimately be handled in an unpredictable way prior to the lysis (extraction) stage.

The present application proposes an interface between a tissue sample carrier which combines the processes of sample dissection and preparation, and the molecular analysis of tissue specimens.

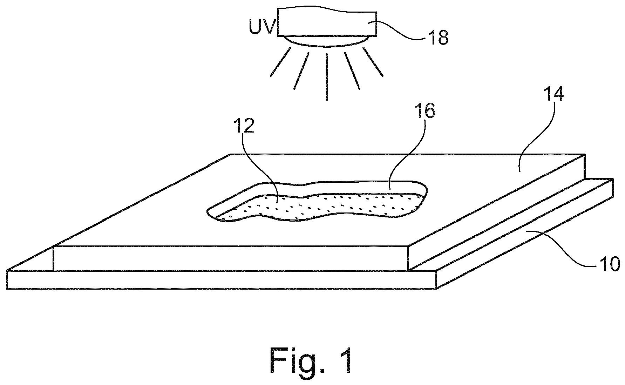

Figure 1 illustrates a technique for sample selection where a tissue slice 12 is provided on top of a sample carrier 10, and a barrier layer 14 having an aperture 16 is deposited on top of the tissue slice 12 and sample carrier 10 by an inkjet printer (not shown), for example. The barrier layer 14 is typically hardened via exposure to ultraviolet light 18. The lateral extent of the aperture 16 is typically chosen to expose a region of interest of the tissue slice 12 through the aperture 16, and to seal a remaining portion of the tissue slice 12. The lateral extent of the aperture may, for example, be selected using a computer imaging software program to identify tumour cells, or alternatively a medical professional may define the lateral extent of the aperture.

In this way, when the sample carrier 10 is submerged in a lysis fluid, or other reagent, only the tissue sample in the region of interest will be exposed to the lysis fluid through the aperture 16. Because the barrier layer 14 exposes the tissue only in the region of interest selected by a pathologist, the sample obtained will have enhanced purity and/or a large number of tumour cells present.

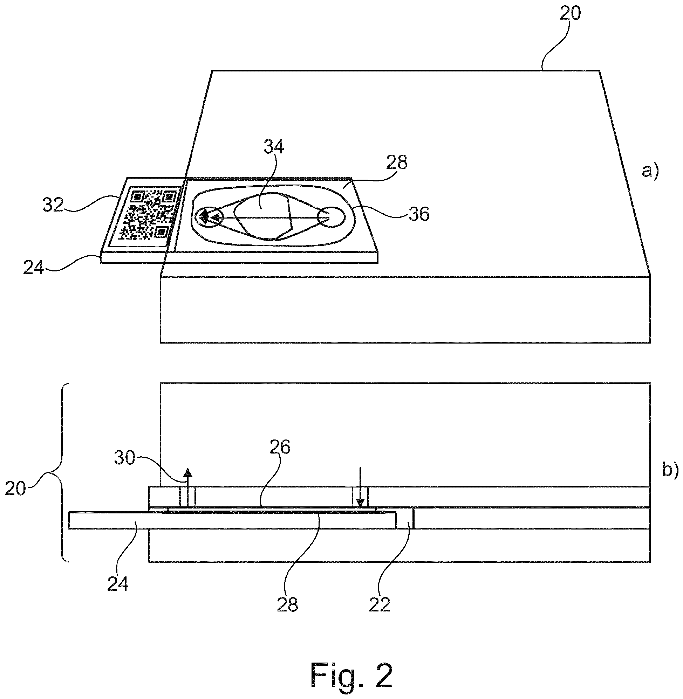

Figure 2b) schematically illustrates a side cut-through view of an example of a microfluidic cartridge 20 for predefined local extraction of biomolecules from biological sample according to the first aspect. The microfluidic cartridge 20 comprises a receptacle 22 for receiving a sample carrier 24 in an analysis position (illustrated). The receptacle 22 comprises a sealable surface 26 having a predefined geometry configured to match to a predefined barrier layer 28 of a sample carrier 24 placed in the analysis position. When the sample carrier 24 is in the analysis position, an atmospherically closed connection is formed between the barrier layer 28 of the sample carrier 24 and the sealable surface 26 of the microfluidic cartridge enclosing a region of interest of biological material in a sealable analysis chamber.

The microfluidic cartridge 20 also comprises an extraction fluid storage chamber for storing an extraction fluid having at least one channel 30 in fluid communication with the sealable analysis chamber. The extraction fluid is usable to release biomolecules from the region of interest of the biological sample.

The microfluidic cartridge 20 also comprises an output portion to enable the transfer of extraction fluid comprising released biomolecules.

Optionally, the receptacle 22 of microfluidic cartridge 20 is provided with a mechanical slot that receives the sample carrier comprising the tissue of interest and the barrier layer.

Optionally, the microfluidic cartridge 20 and/or the sample carrier 24 may comprise a barcode detection unit or "QR" code detection unit to verify the correctness of the tissue sample introduced.

Optionally, receptacle 22 of the microfluidic cartridge comprises a guide, and/or a mechanical reference, to ensure that the sample carrier is introduced into the analysis position correctly and completely.

Optionally, a soft seal may be present on the surface of the receptacle 22 that is brought into contact with the barrier layer present on the sample carrier. The soft seal functions to atmospherically seal an aperture of the barrier layer exposing region of interest of tissue such that fluid may be introduced into the aperture region during predefined local extraction of biomolecules.

At least one channel providing fluid access to the sealed region comprising the region of interest of tissue is provided to enable extraction buffer to be pumped into the sealed region.

Optionally, the microfluidic cartridge 20 comprises a fluid actuation mechanism, such as a piston-driven actuation, or a peristaltic pump actuation, or other microfluidic pumps.

Optionally, the microfluidic cartridge 22 comprises a heater to enable the extraction buffer to be preheated. The heater may be provided in, or around, an extraction buffer storage chamber, on one or both sides in proximity to the sealed region of the sample carrier, and/or in the vicinity or around the fluid transport channel between an extraction fluid storage chamber and the sealed region.

Optionally, microfluidic cartridge 22 may comprise a fluid receiving chamber enabling buffer fluid comprising extracted biomolecules to be collected. Optionally, the fluid receiving chamber comprises processing means process the buffer fluid and extracted biomolecules according to a selected molecular assay protocol. It will be appreciated that a wide range of molecular assay protocols can be applied. For example, a sample purification means is provided in the fluid receiving chamber enabling sample purification by binding to and eluting from a specific binding medium on a filter, or on microbeads. Optionally, after purification the eluted material can be amplified by mixing with one or more of primers, probes, and enzymes mixed in a dedicated buffer.

The presence of the target molecule in the extracted buffer can be identified, for example, by measuring fluorescence properties or electrical properties of the extracted fluid during, or after amplification.

It will be appreciated that the output portion may, in one option, be equipped with an analyzer to perform the above discussed extraction and analysis steps in their entirety. Alternatively or in combination, the output portion may comprise a fluid channel to enable the extracted buffer fluid to be transferred to a sample analysis system that the microfluidic cartridge 20 has been inserted into. Both approaches have advantages. For example, integrating the extraction and analysis inside the microfluidic cartridge 20 means that a complete analysis of the region of interest of a tissue sample can be provided without large and cumbersome analysis machines. However, the transfer of the buffer fluid outside of the microfluidic cartridge 20 into a sample analysis system may enable a more sensitive test to be applied to the buffer fluid using a more advanced sample analysis system, and/or an independent quality control test.

It will be appreciated that Figure 2b) shows a microfluidic cartridge 20 with a sample carrier 24 mounted in an analysis position of the microfluidic cartridge 20.

Figure 2a) schematically illustrates an isometric translucent view of the microfluidic cartridge 20 described above, when a sample carrier 24 is fitted inside its analysis position. The sample carrier 24, shown with an optional "QR" code 32, can be seen to comprise a tissue region of interest 34 exposed by a barrier layer 28 which has been selectively applied to the surface of the sample carrier 24. When the sample carrier 24 is in the analysis position of the microfluidic cartridge 20, a seal 36 is formed by the interaction of the sealable surface 26 of the microfluidic cartridge 20, and the predefined barrier layer 28 of the sample carrier 24.

The seal fully surrounds the region of interest 34 of the exposed region of the tissue sample, thus providing a chamber for the predefined local extraction of biomolecules in-between the surface of the sample carrier 24 having the barrier layer and the region of interest, and the sealable surface of the microfluidic cartridge 20, when sample carrier 24 is in an analysis position of the microfluidic cartridge 20.

The sealable surface 26 of the microfluidic cartridge 20 is optionally formed from a resilient substance selected from the group of rubber, foam rubber, silicon rubber, polyolefin, thermoplastic polymer, and EDPM. These substances may be provided in a predefined geometry matching to a sample carrier barrier layer geometry. This means that when a sample carrier is placed in an analysis position of a receptacle 22 of the microfluidic cartridge 20, the sealable surface 26 resiliently surrounds the region of interest on the sample carrier to provide an atmospheric seal.

In other words, the interaction of the sealable surface 26 and the barrier layer 28 around the region of interest creates a sealed analysis chamber. It should be understood that the analysis chamber is in fluid communication with input and output ports for the delivery of analysis fluid and the removal of extracted biomolecules, but that when the sample carrier 24 is in the analysis position, fluid cannot escape outside of the sealed analysis chamber except via the output port or ports.

Optionally, portions of the receptacle 22 and/or the microfluidic cartridge 20 are movable, to enable the sample carrier to be placed in an analysis position of the microfluidic cartridge 20. In a simple example, the microfluidic cartridge 20 is provided as hinged body having a first and second member connected at a hinge. An inner surface of the first member may comprise the sealable surface 26, and optionally mechanical means to ensure a sample carrier is guided into the correct position. In use, the microfluidic cartridge 20 is opened at its hinge, a sample carrier is inserted along the guide means, and then the first and second members of the microfluidic cartridge are closed, with the sample carrier being fixedly held in an analysis position of the receptacle 22 of the microfluidic cartridge 20. The hinged closing action brings the inner surface of the first member comprising the sealable surface 26 in a predefined geometry into physical engagement with the surface of the barrier layer of the sample carrier, thus forming an atmospheric seal around a region of interest exposed in an aperture of the barrier layer. The microfluidic cartridge 20 in this state would be prepared for sample extraction to commence. Of course, other microfluidic cartridge actuation approaches for sealing a sample carrier in an analysis position may be provided, such as a microfluidic cartridge 20 which may be split entirely into two portions, and clipped around the sample carrier 24. Alternatively, a one-piece microfluidic cartridge could be provided enabling the sliding of the sample carrier into the receptacle along guide rails.

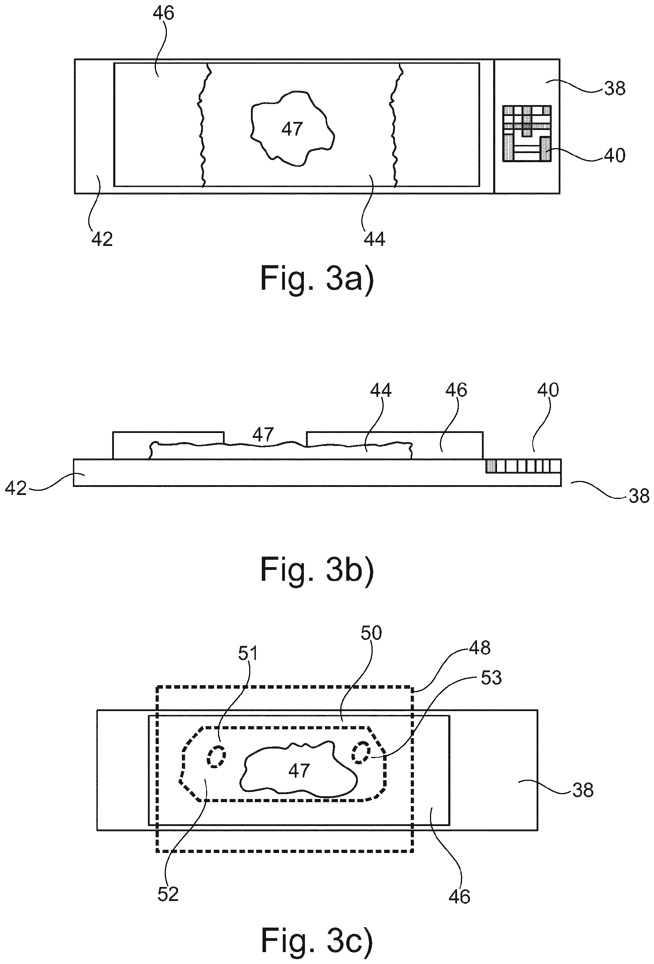

Figure 3a) illustrates a top-down view of a sample carrier 38 in accordance with the second aspect, optionally configured to be received into a mechanical slot of a receptacle in a microfluidic cartridge 22. The sample carrier optionally comprises a "QR" code 40 for identification of tissue samples. A base portion 42 of the sample carrier, optionally made of glass, provides a substantially planar surface for the support of a tissue sample 44. A barrier layer 46, optionally of an ultraviolet curable polymer layer, is applied such that it covers part of the base portion 42 of the sample carrier 38, but exposes a region of interest of the tissue sample 47.

Figure 3b) illustrates a side-view of the sample carrier 38.

Figure 3c) illustrates a top-down view of the sample carrier 38, with dotted lines 48 and 50 illustrating the outer and inner extent of the predefined geometry of the sealable surface of the receptacle 22 of the microfluidic cartridge 20, relative to the sample carrier 38, when the sample carrier 38 is inserted into the receptacle 22. The sealable surface defined by dotted lines 48 and 50 is a feature of the microfluidic cartridge, and would remain the same for any shape of region of interest 47. The software used to define the barrier layer prior to, for example, inkjet printing the barrier layer onto the sample slide has knowledge of the various predefined geometries of the sealable surface of different types of microfluidic cartridge, and is able to print the barrier layer such that the region of interest 47 so formed, does not extend into the region in-between the dotted lines 48 and 50 (the geometry defining the sealable surface), otherwise the fluid-tightness of the seal would be harmed, for example.

It will be seen that an inner cavity 52 is formed by the interaction of the sealable surface of the receptacle 22 and the barrier layer 46 of the sample carrier 38. Exposed inside the inner cavity is the aperture providing fluid access to the tissue sample 47. In other words, the inner cavity formed by the sealable surface of the receptacle and the barrier layer of the sample carrier provides a sealable analysis chamber fully enclosing a region of interest of a tissue sample, when the sample carrier 38 is fitted in an analysis position of receptacle 22 of the microfluidic cartridge 20 according to the first aspect. The dotted lines 51 and 53 represent, respectively, the location of fluid input and output ports of a fitted microfluidic cartridge when the sample carrier 38 is fitted to the microfluidic cartridge in an analysis position.

Typically, when a sample carrier is inserted into the receptacle 22, in an analysis position of the microfluidic cartridge 20, the volume of the sealed analysis chamber is preferably in the range of 10 to 1000 µL. However, the volume of the sealed analysis chamber may optionally be in the ranges of 10 to 2000 µL, 10 to 3000 µL, 10 to 4000 µL, 10 to 5000 µL, 10 to 6000 µL, 10 to 8000 µL, 10 to 10,000 µL 10 to 4000 µL, 10 to 3000 µL, or 10 to 2000 µL, for example.

The sample carrier may, for example, be constructed from a glass slide, upon which a barrier layer is subsequently deposited. Alternatively, a polymer, or laminated polymer on a glass slide may be used, for example.

The barrier layer is optionally provided as a layer of cured chemical applied to the sample carrier and the first area of the biological sample. Typical cured chemicals used to form the barrier layer are UV-cured resins, acrylates, methacrylates, enols, epoxides, and acrylamides.

It will be appreciated that the degree of integration of the various sample preparation steps can be chosen based on preferences.

Optionally, the microfluidic cartridge 20 comprises an extraction fluid storage chamber comprising extraction buffer, and a reservoir connected to the upper portion for receiving the extracted material, and fluid actuation means and the temperature control element open bracket heater). The extracted sample can be transferred from such a cartridge to another system such as a pipetting robot, or another cartridge having integrated sample processing via the output portion enabling the transfer of extraction fluid. The output portion may optionally be a manual pipetting port.

Optionally, sample preparation steps up to and including biomolecules detection are housed in the microfluidic cartridge 20 with all reagents inside the microfluidic cartridge 20.

Optionally, the detection of target molecules is integrated into the cartridge. Thus, no manual steps are required after the presentation of the sample carrier into the receptacle 22 of the microfluidic cartridge 20. Optionally, the output portion of the microfluidic cartridge 20 comprises an interface to present the extracted sample to a pipetting robot, in a manner compatible with a well plate. For example, the microfluidic cartridge 20 may comprise surface having fluid reservoirs connected to the output portion at positions complying with a given standard for pipetting robots.

The operation of the microfluidic cartridge for predefined local extraction will now be explained.

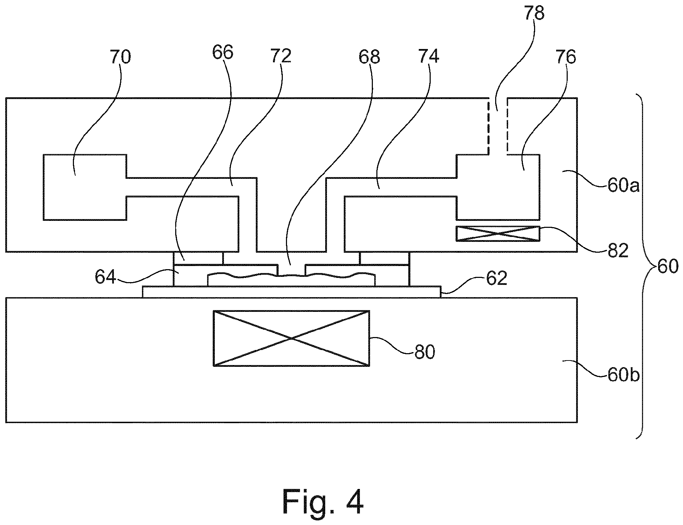

Figure 4 illustrates an exemplary schematic cross-sectional view of a receptacle of a microfluidic cartridge 60 discussed in an operational example, comprising a base portion 60b and an upper portion 60a. A sample carrier 62 has been placed into an analysis position inside the receptacle, and the upper portion 60a and/or the base portion 60b have been moved together such that the barrier layer 64 on the sample carrier 62 is brought into sealing contact with a sealable surface 66 of the upper portion 60a of the microfluidic cartridge 60, thus forming a sealed analysis chamber 68. Also shown is an extraction fluid storage chamber 70 (shown empty in Figure 4) connected so as to be in fluid communication, via a fluid channel 72, with the sealed analysis chamber 68. Furthermore, an output fluid channel 74 is shown in fluid communication with the sealed analysis chamber 68 and an output portion 76. In the optional embodiment of Figure 4, the output portion 76 further comprises a fluid analysis chamber for receiving extraction fluid containing locally extracted biomolecules, and an output fluid channel 78 in fluid communication with the output of the microfluidic cartridge 60. Optionally, the output fluid channel 78 may be provided with a pipetting teat or valve. Optionally, a heater 80, in thermal contact with the sample carrier 62, is provided in the base portion 60b of the microfluidic cartridge 60b, although it will be appreciated that the heater could also be provided in the upper portion 60a of the microfluidic cartridge 60, provided thermal energy can be communicated to the sealed analysis chamber 68.

Optionally, an analyser is provided in fluid communication with the fluid analysis chamber comprising, for example, a sample purifier using a filter or microbeads, a biomolecular amplifier configured to apply a primer to the released biomolecules, and fluorescence and/or electrical analysers. Thus, an initial reading of biomolecules may be taken using the analyser 82, and locally extracted fluid may be removed from the fluid analysis chamber 76 via the output fluid channel 78 for quality control.

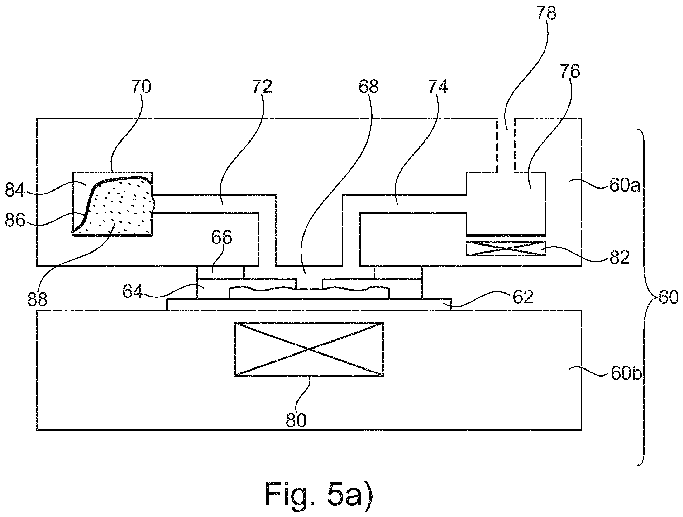

Figure 5a) illustrates the receptacle of the microfluidic cartridge 60 at the beginning of a predefined local extraction of biomolecules. The extraction fluid storage chamber 70 contains a reagent, in this example a lysing fluid 88 suitable for the test to be performed held in place by a pump. In the illustrated case, the pump is a microfluidic diaphragm 86, in which an air pocket 84 is gradually filled with positive-pressure gas, thus progressively expelling the lysing fluid 88 into the fluid channel 72, and thence into the sealed analysis chamber 68. Optionally, actuators for providing the positive-pressure gas are comprised within the microfluidic cartridge 60. Alternatively, or in combination, the source of positive-pressure gas may be provided by an external analysis system into which the microfluidic cartridge 60 has been inserted. Optionally, the fluid actuation may be performed by a piston, or a peristaltic pump, or other techniques known in the art.

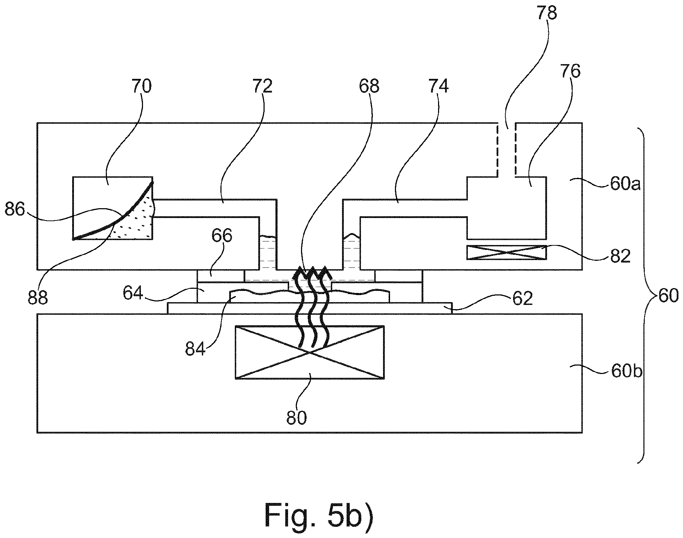

Figure 5b) illustrates the receptacle of the microfluidic cartridge 60 during a predefined local extraction of biomolecules. The extraction fluid storage chamber 70 is partially evacuated of lysing fluid 88, which has flowed through the fluid channel 72 and is substantially contained inside the sealable analysis chamber. The fluid-tight seal formed between the barrier layer 64 of the sample carrier 62 and the sealable surface 66 of the upper portion 60a of the microfluidic cartridge 60 whilst the sample carrier 62 is in the analysis position of the receptacle ensures that the lysing fluid (and/or other reagents) does not escape from the sealable analysis chamber into the receptacle. The barrier layer 64 comprises an aperture, enabling a region of interest of a portion of biological material 84 to come into fluid contact with the lysing fluid (and/or other reagents), and thus for biomolecules to be extracted from a predefined region of interest of the biological material on the sample carrier 62. Optionally, heater 80 is activated to enhance a lysis or other reagent reaction with the biological material.

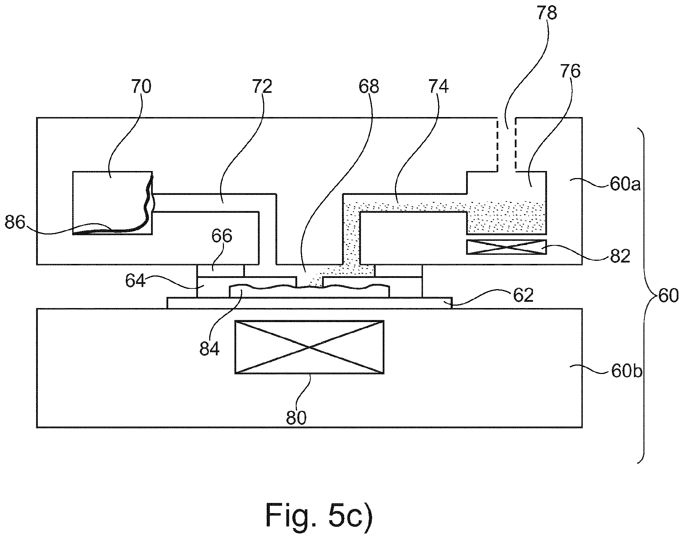

Figure 5c) illustrates the receptacle of the microfluidic cartridge 60 after a predefined local extraction of biomolecules. The extraction fluid storage chamber 70 is optionally substantially evacuated of extraction fluid. Alternatively, the extraction fluid storage chamber 70 may retain an amount of reagent that is not required for an extraction. Through the action of positive-pressure gas pumping, suction, or the like, the fluid analysis chamber of an output portion 76 contains the lysing fluid (reagent) now containing biomolecules extracted from a predefined area of the biological material 84 in the previous step. As discussed previously, the lysing fluid (reagent) may be analysed by an analyser 82 comprised within the microfluidic cartridge 60. In this case, the microfluidic cartridge 60 may communicate a test result to a microfluidic testing system that the microfluidic cartridge 60 is plugged into using an analogue or digital electrical signal. In other words, the microfluidic cartridge 60 may comprise the analysis electronics for identifying locally extracted biomolecules. Alternatively or in combination, a portion of the lysing fluid (reagent) containing biomolecules may be extracted from the microfluidic cartridge 60 via output fluid channel 78 by a fluidic analysis system, or by pipetting, for example, for a further analysis step, and/or a quality control step.

Optionally, a fluidic recirculation channel (not shown) may be provided between the extraction fluid storage chamber 70 and the output portion, to enable the recirculation of reagent.

Although a microfluidic cartridge 60 has previously been illustrated and discussed with reference to one extraction fluid storage chamber 70, and/or one fluid analysis chamber 76, it will be appreciated that this is a simple example. For example, a microfluidic cartridge 60 can optionally be provided in accordance with aspects of the invention having a plurality of fluid storage chambers each in fluid communication with the sealable analysis chamber 68. A microfluidic cartridge 60 can optionally be provided in accordance with aspects of the invention having a plurality of fluid analysis chambers in fluid communication with the sealable analysis chamber 68. Furthermore, a system of microfluidic valves can, for example, be provided along with a control system to sequence the application of a plurality of different reagents during the predefined local extraction of biomolecules from a biological sample according to test protocols known in the field of biomolecular extraction.

Optionally, tissue deparaffination means may be integrated into the microfluidic cartridge 60, such that a tissue deparaffination step of the region of interest may be performed before the application of reagent to the region of interest.

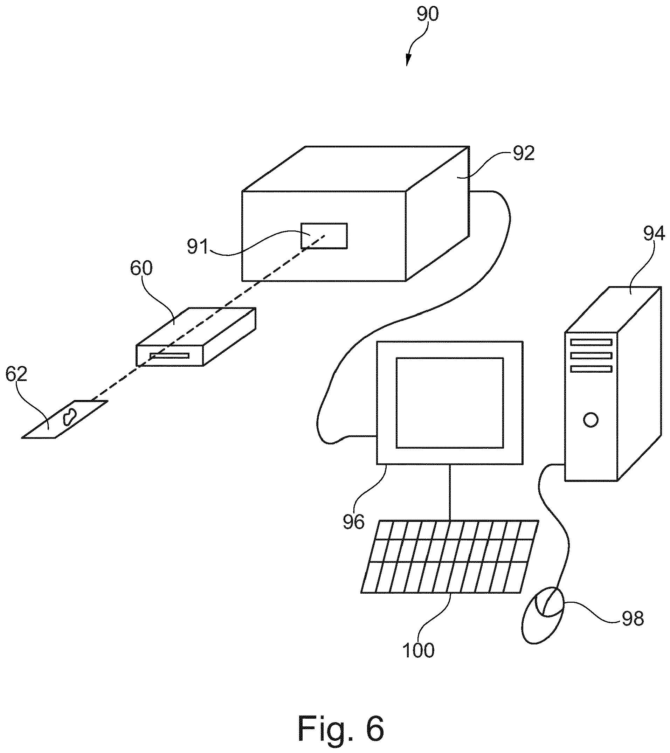

Figure 6 illustrates a microfluidic analysis system 90. The system comprises an extraction fluid analyser 92 and/or electronic interface configured to interface with the output portion of a microfluidic cartridge 60. The microfluidic cartridge 60 is configured to accept a sample carrier 62. The system further comprises a data processor 94 configured to generate analysis data based on an output of the extraction fluid analyser 92 and/or electronic interface. The system further comprises an output display 96 configured to display microfluidic analysis results to a user. Optionally, the data processor 94 is a personal computer comprising a mouse 98 and a keyboard 100.

It will be appreciated that the microfluidic analysis system may comprise an extraction fluid analyser which obtains biomolecules extracted from a biological sample via a fluid output channel of a microfluidic cartridge 60. As a microfluidic cartridge 60 containing a sample carrier 62 is engaged into a cartridge receptacle 91 of the extraction fluid analyser, a fluidic and/or electrical connection is made between the microfluidic cartridge 60 and the extraction fluid analyser 92 to enable extraction and analysis of reagent from the microfluidic cartridge. Alternatively, or in combination, the microfluidic answer system may comprise an electronic interface configured to read result data of a reagent from an analogue and/or digital interface of a microfluidic cartridge 60. The results may be read from the microfluidic cartridge 60 before, during, and/or after a predefined local extraction of biomolecules inside the microfluidic cartridge 60.

According to a fourth aspect, there is provided method for predefined local extraction of biomolecules from a biological sample. The method comprises:
a) receiving a sample carrier into an analysis position of a receptacle of a microfluidic cartridge, wherein the receptacle comprises a sealable surface having a predefined geometry configured to match to a predefined geometry of a barrier layer of a sample carrier placed in the analysis position, such that when the carrier is in the analysis position, an atmospherically closed connection is formed between the barrier layer and the sealable surface, enclosing an aperture in the barrier layer around a region of interest of biological material in a sealed analysis chamber; and
b) transferring an extraction fluid into the sealed analysis chamber , wherein the extraction fluid is used to release biomolecules from the region of interest of the biological sample not covered by the barrier layer; and
c) outputting extraction fluid comprising biomolecules released from the region of interest of the biological sample not covered by the barrier layer to an output portion of the microfluidic cartridge.

Optionally, the method comprises:
b1) heating the sealed analysis chamber thereby to heat the extraction fluid in the sealed analysis chamber.

According to a fifth aspect, there is provided a kit of parts. The kit of parts comprises:
- a microfluidic cartridge as defined according to the first aspect and its embodiments, and
- a sample carrier according to the second aspect and its embodiments.

It should be noted that embodiments of the invention are described with reference to different subject-matter. In particular, some embodiments are described with reference to method-type features, whereas other embodiments are described with respect to apparatus-type features. A person skilled in the art will gather from the above, and following description, that, unless otherwise notified, in addition to any combination of features belonging to one type of subject-matter, also any combination of features belonging to one type of subject-matter, also any combination between features relating to different subject-matter is considered to be disclosed within this application. All features can be combined to provide a synergetic effect, which is more than the simple summation of the features.

Whilst the invention has been illustrated and described in detail in the drawings and the foregoing description, such illustration and description are to be considered to be illustrative or exemplary, and not restrictive. The invention is not limited to the disclosed embodiments. Other variations to the disclosed embodiments can be understood, and effected, by those skilled in the art in practicing the claimed invention, from a study of the disclosure in the drawings, the description, and the dependent claims.

In the claims, the word "comprising" does not exclude other elements or steps. The indefinite article "a" or "an" does not exclude a plurality. A single processor, or other unit, may fulfil the functions of several items recited in the claims. The mere fact that certain measures are recited in mutually different dependent claims does not indicate that a combination of these measures cannot be used to advantage.

Any reference signs in the claims should not be construed as limiting the scope of the claims.

## Claims

1. A microfluidic cartridge (20) for predefined local extraction of biomolecules from a biological sample (34), comprising:
- a receptacle (22) for receiving a sample carrier (24) in an analysis position, wherein the receptacle comprises a sealable surface (26) having a predefined geometry configured to match to a predefined barrier layer of a sample carrier placed in an analysis position, such that when the sample carrier is in the analysis position, an atmospherically closed connection is formed between the barrier layer and the sealable surface enclosing an aperture in the barrier layer around a region of interest of biological material in a sealable analysis chamber;
- an extraction fluid storage chamber for storing an extraction fluid having at least one channel (30) in fluid communication with the sealable analysis chamber, wherein the extraction fluid is used to release biomolecules from the region of interest of the biological sample, and
- an output portion to enable the transfer of extraction fluid comprising released biomolecules.

2. The microfluidic cartridge (20) according to claim 1, further comprising
- a fluid analysis chamber in fluid communication with the output portion;
wherein the fluid analysis chamber is in fluid communication with one or more of the analyzers selected from the group of: a sample purifier using a filter or microbeads, a biomolecular amplifier configured to apply a primer to the released biomolecules, and fluorescence and/or electrical analysers.

3. The microfluidic cartridge (20) according to claim 1,
wherein the fluid storage chamber and/or the fluid receiving chamber are in fluid connection with a port or a plurality of ports located on an external surface of the microfluidic cartridge, to enable extraction fluid comprising released biomolecules to be transferred for external analysis.

4. The microfluidic cartridge (20) according to one of the preceding claims,
wherein the sealable surface (26) of the receptacle is formed from a resilient substance selected from the group of rubber, foam rubber, silicon rubber, polyolefin, thermoplastic polymer, and EDPM.

5. The microfluidic cartridge (20) according to one of the preceding claims,
wherein the sealed analysis chamber has an internal volume in the range of 10-1000 µL.

6. The microfluidic cartridge (20) according to one of the preceding claims,
wherein the receptacle further comprises:
- a first actuator causing the sealable surface to be configurable into a first retracted position, and a first sealed position, and wherein the sealed extraction chamber is formed when the first actuator is actuated from the first retracted position into the first sealed position, to thus cause the sealable surface to move into a sealing engagement with a barrier layer of a sample carrier.

7. The microfluidic cartridge (20) according to one of the preceding claims,
wherein the receptacle further comprises:
- a sample carrier actuator causing a sample carrier, when held in the receptacle, to be configurable into a second retracted position, and a second sealed position, and wherein the sealed extraction chamber is formed when the sample carrier actuator is actuated from the second retracted position into the second sealed position, to thus cause a barrier layer of a sample carrier to move into a sealing engagement with the sealable surface of the receptacle.

8. The microfluidic cartridge (20) according to one of the preceding claims,
wherein the microfluidic cartridge further comprises a microfluidic cartridge carrier ID configured to identify the microfluidic cartridge.

9. A sample carrier (24) for local extraction of biomolecules from a biological sample (34) comprising:
- a sample carrier (24) configured to hold a biological sample, and
- an impermeable barrier layer (28) covering a portion of the biological sample, and exposing a region of interest of the biological sample through an aperture in the barrier layer,
wherein the barrier layer on the sample carrier has a predefined geometry which is configured to match to a predefined geometry of a sealable surface of a microfluidic cartridge such that when the sample carrier is in an analysis position of a microfluidic cartridge for predefined local extraction of biomolecules from a biological sample, a sealed analysis chamber is formed containing the region of interest of the biological sample.

10. The sample carrier (24) according to claim 9,
wherein the barrier layer (28) of the sample carrier is formed from a layer of chemical applied to the sample carrier and a first area of the biological sample, and subsequently cured.

11. The sample carrier (24) according to claim 10,
wherein the cured chemical is selected from the group of: UV-cured resins, acrylates, methacrylates, enols, epoxides, and acrylamides.

12. A microfluidic analysis system (90), comprising:
- a cartridge receptacle (91) for receiving a microfluidic cartridge (60) according to one of claims 1 to 8;
- an extraction fluid analyser (92) and/or electronic interface configured to interface with the output portion of the microfluidic analysis system;
- a data processor (94) configured to generate analysis data based on an output of the extraction fluid analyser and/or the electronic interface; and
- an output display (96) configured to display microfluidic analysis results to a user.

13. A method for predefined local extraction of biomolecules from a biological sample, comprising:
a) receiving (102) a sample carrier into an analysis position of a receptacle of a microfluidic cartridge, wherein the receptacle comprises a sealable surface having a predefined geometry configured to match to a predefined geometry of a barrier layer of a sample carrier placed in the analysis position, such that when the carrier is in the analysis position, an atmospherically closed connection is formed between the barrier layer and the sealable surface, enclosing an aperture in the barrier layer around a region of interest of biological material in a sealed analysis chamber; and
b) transferring (104) an extraction fluid into the sealed analysis chamber, wherein the extraction fluid is used to release biomolecules from the region of interest of the biological sample not covered by the barrier layer; and
c) outputting (106) extraction fluid comprising biomolecules released from the region of interest of the biological sample not covered by the barrier layer to an output portion of the microfluidic cartridge.

14. The method according to claim 13, further comprising:
b1) heating the sealed analysis chamber thereby to heat the extraction fluid in the sealed analysis chamber.

15. A kit of parts, comprising:
- a microfluidic cartridge as defined according to one of claims 1 to 8, and
- a sample carrier according to one of claims 9 to 11.
